# FASCICULE DE BREVET EUROPEEN

(11) **EP 3 357 449 B1**
(45) Date de publication et mention de la délivrance du brevet: **06.02.2019**
(21) Numéro de dépôt: 18158188.5
(22) Date de dépôt: 09.12.2013
(51) Int. Cl.: A61C 1/05, A61C 1/12, A61B 17/16

(54) **TURBINE POUR UNE PIÈCE À MAIN DENTAIRE OU CHIRURGICALE À AIR COMPRIMÉ**
TURBINE FÜR EIN DRUCKLUFTBETRIEBENES ZAHNÄRZTLICHES ODER CHIRURGISCHES HANDSTÜCK
TURBINE FOR A COMPRESSED-AIR DENTAL OR SURGICAL HANDPIECE

(43) Date de publication de la demande: 08.08.2018
(62) Demande divisionnaire de: 13196222.7
(73) Titulaire: Bien-Air Holding SA, 2500 Bienne 6 (CH)
(72) Inventeur: Farine, Laurent, 2740 Moutier (CH)
(74) Mandataire: ICB SA

(56) Documents cités:
- WO-A1-2006/053153
- CN-U- 202 714 934
- DE-A1- 19 504 298
- GB-A- 1 002 596
- US-A- 3 893 242

## Description

La présente invention concerne une turbine pour une pièce à main dentaire ou chirurgicale à air comprimé. La présente invention concerne en particulier une turbine pour une pièce à main à air comprimé présentant une puissance accrue.

La présente invention concerne le domaine des pièces à main à usage chirurgical ou dentaire. Il existe deux familles de pièces à main : les contre-angles qui sont équipés d'un moteur électrique et les pièces à main qui comprennent une turbine entraînée par de l'air comprimé.

On s'intéresse ici aux pièces à main à air comprimé. Très schématiquement, ces pièces à main à air comprimé comprennent une tête à l'intérieur de laquelle est logée une turbine. Cette turbine comprend un conduit d'amenée de l'air comprimé et une roue mobile munie sur sa périphérie d'une pluralité d'aubes régulièrement espacées. Le conduit d'amenée sert à forcer sur les aubes de la turbine un flux d'air comprimé dont l'énergie pneumatique se transforme en énergie cinétique lorsque le flux d'air comprimé frappe les aubes de la roue mobile.

La figure 1 annexée à la présente demande de brevet est une vue en perspective et en coupe selon un plan horizontal coupant longitudinalement le manche et la tête d'une pièce à main à air comprimé selon l'art antérieur. Désignée dans son ensemble par la référence numérique générale 1, cette pièce à main comprend un manche 2 qui se raccorde à une tête 4 à l'intérieur de laquelle est logée une turbine 6. La turbine 6 comprend une roue mobile 8 depuis une périphérie 10 de laquelle s'étend une pluralité d'aubes 12 qui définissent un diamètre extérieur 14 de la roue mobile 8.

La pièce à main 1 comprend également des moyens d'injection ayant pour fonction de diriger sur les aubes 12 de la roue mobile 8 un flux d'air comprimé dont l'énergie pneumatique se transforme en énergie cinétique lorsque le flux d'air comprimé frappe les aubes 12 de la roue mobile 8. Les moyens d'injection de l'air comprimé se composent d'un conduit 16 d'amenée de l'air comprimé qui est usiné selon des techniques conventionnelles dans le manche 2 de la pièce à main 1. Par ailleurs, un conduit 18 d'échappement de l'air est également usiné par des techniques classiques dans le manche 2 de la pièce à main 1, sensiblement parallèlement et à distance du conduit 16 d'amenée de l'air comprimé.

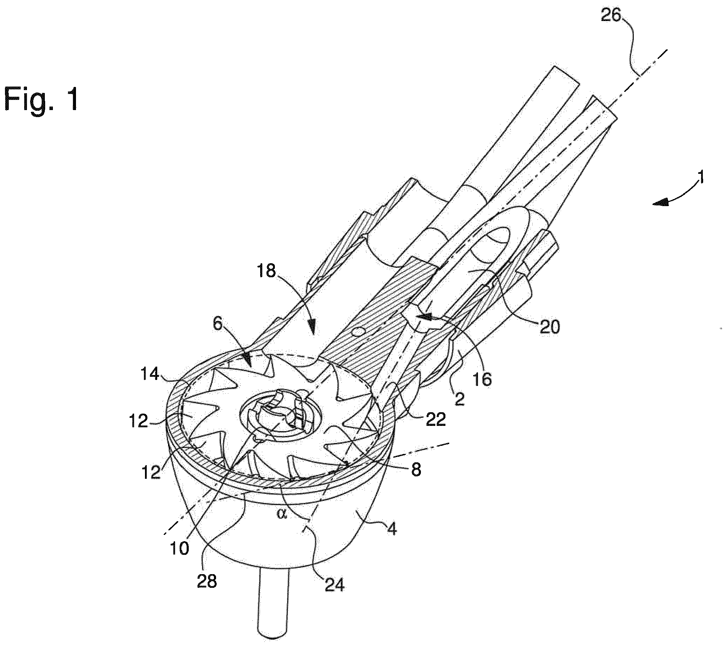

On voit, à l'examen de la figure 1, que le conduit 16 d'amenée de l'air comprimé se compose d'un premier et d'un second conduit rectilignes, respectivement 20 et 22, disposés dans le prolongement l'un de l'autre, l'axe longitudinal de symétrie 24 du second conduit rectiligne 22 s'étendant légèrement de biais par rapport à l'axe longitudinal de symétrie 26 du manche 2 de la pièce à main 1 et formant un angle g non-nul avec la tangente 28 au diamètre extérieur 14 de la roue mobile 8. On comprend qu'il est difficilement envisageable d'usiner le conduit 16 d'amenée de l'air comprimé selon une direction qui tendrait à se rapprocher de la tangente 28 au diamètre extérieur 14 de la roue mobile 8, au risque de percer le conduit 18 d'échappement de l'air ou de devoir réduire le diamètre de ce conduit 18.

Le document GB 1,002,596 A divulgue une turbine selon le préambule de la revendication 1.

Un problème récurrent qui se pose aux constructeurs de pièces à main à air comprimé réside dans l'efficacité de la conversion entre l'énergie pneumatique de l'air comprimé et l'énergie cinétique de la roue mobile de la turbine. En effet, de l'efficacité de cette conversion dépend la puissance mécanique que peut fournir la pièce à main à air comprimé. Cette efficacité de conversion est notamment étroitement liée à la chute de pression entre la pression à laquelle se trouve l'air comprimé qui pénètre dans la tête de la pièce à main, et la pression de l'air qui s'échappe de la tête de la pièce à main après que l'air comprimé a frappé les aubes de la turbine. En effet, plus cette chute de pression est importante, meilleure est la conversion entre l'énergie pneumatique et l'énergie cinétique. L'efficacité de conversion entre l'énergie pneumatique et l'énergie cinétique est également d'autant meilleure que l'écoulement de l'air se fait avec le moins de turbulences et donc le moins de pertes possible. On cherche également à réduire les bruits de fonctionnement des pièces à main à air comprimé et à éviter autant que possible que l'air comprimé destiné à actionner la turbine ne fuie dans la bouche du patient

La présente invention a pour but de répondre aux objectifs mentionnés ci-dessus ainsi qu'à d'autres encore en procurant notamment une turbine pour une pièce à main fournissant une puissance mécanique plus élevée.

A cet effet, la présente invention concerne une turbine pour une pièce à main à air comprimé à usage dentaire ou chirurgical, la turbine comprenant une roue mobile depuis une périphérie de laquelle s'étendent une pluralité d'aubes qui définissent un diamètre extérieur de la roue mobile et sur lesquelles tombe un flux d'air comprimé, chacune des aubes se composant d'une première surface qui s'étend depuis le diamètre extérieur de la roue mobile et qui mène jusqu'à une seconde surface qui s'étend selon un rayon de la roue mobile, les aubes étant associées par paires de manière étagée, caractérisée en ce que chaque aube est séparée de l'aube à laquelle elle est associée par une échancrure qui s'étend dans la direction du flux d'air comprimé, de sorte que le flux d'air comprimé se partage en deux parts égales au moment où il atteint l'échancrure, l'échancrure permettant de réduire un temps de transit du flux d'air comprimé d'une aube à l'autre.

Selon un mode préféré de réalisation de l'invention, la première surface s'étend depuis le diamètre extérieur de la roue mobile jusqu'à un point du rayon de la roue mobile à partir duquel s'étend radialement la seconde surface jusqu'au diamètre extérieur de la roue mobile.

Enfin, selon une autre caractéristique de l'invention, les aubes sont associées par paire de manière étagée, chaque aube étant séparée de son aube correspondante par une échancrure qui s'étend dans la direction du flux d'air comprimé, de sorte que le flux d'air se partage en deux parts égales au moment où il atteint l'échancrure. Cette échancrure a pour but de réduire le temps de passage d'une aube à l'aube suivante afin de limiter les pertes de puissance mécanique de la turbine.

La première surface conduit vers la seconde surface l'air comprimé qui sort des moyens d'injection. Comme la seconde surface s'étend selon un rayon de la roue mobile et que le flux d'air comprimé est canalisé selon une direction tangente au diamètre extérieur de la roue mobile, le flux d'air comprimé tombe perpendiculairement sur la seconde surface de l'aube puis fait un demi-tour. Or, la puissance mécanique délivrée par une turbine est d'autant plus grande que l'angle de déviation de l'air comprimé est important. Dans le cas de la présente invention, l'angle de déviation de l'air comprimé est de 180°, ce qui correspond à un optimum de conversion entre l'énergie pneumatique de l'air comprimé et l'énergie cinétique de la turbine.

La pièce à main à air comprimé comprend un manche qui se raccorde à une tête à l'intérieur de laquelle est logée une turbine, la turbine comprenant une roue mobile depuis une périphérie de laquelle s'étendent une pluralité d'aubes qui définissent un diamètre extérieur de la roue mobile, la pièce à main comprenant également des moyens d'injection ayant pour fonction de diriger sur les aubes de la roue mobile un flux d'air comprimé dont l'énergie pneumatique se transforme en énergie cinétique lorsque le flux d'air comprimé frappe les aubes de la roue mobile, la pièce à main comprenant de plus un conduit d'échappement ménagé dans le manche de la pièce à main et par lequel s'échappe l'air après avoir frappé les aubes de la roue mobile, la pièce à main étant caractérisée en ce que le conduit d'échappement se trouve au moins en regard de la zone dans laquelle l'air comprimé frappe les aubes de la roue mobile.

La section du conduit d'échappement s'étend sur au moins la hauteur des aubes de la roue mobile.

Les moyens d'injection de l'air comprimé sont ménagés dans la paroi du manche de la pièce à main.

Dans la pièce à main à air comprimé, le retour de l'air comprimé après qu'il ait frappé les aubes de la turbine est grandement facilité, ce qui améliore la conversion entre l'énergie pneumatique de l'air comprimé et l'énergie cinétique de la turbine en favorisant la chute de pression entre le moment où l'air comprimé pénètre dans la tête de la pièce à main et le moment où l'air comprimé en ressort. On comprend en effet que, tandis qu'une partie de l'air comprimé contourne la turbine et entraîne celle-ci, l'autre partie de l'air comprimé ricoche sur les aubes de la turbine et crée des perturbations dans la tête de la pièce à main. Or, ces perturbations nuisent considérablement à l'efficacité de conversion entre énergie pneumatique et énergie cinétique. En disposant d'agencer le conduit d'échappement au moins en regard de la zone dans laquelle l'air comprimé frappe les aubes de la roue mobile, on favorise ainsi l'échappement de l'air qui ricoche sur les aubes de la turbine et permet donc de réduire sensiblement les perturbations dans la tête de la turbine.

Les moyens d'injection de l'air comprimé se présentent sous la forme d'un insert à l'intérieur duquel est ménagé un conduit d'amenée du flux d'air comprimé et qui est rapporté à l'intérieur du manche de la pièce à main.

L'insert est disposé à l'intérieur du conduit d'échappement.

L'insert est monté de manière coaxiale à l'intérieur du conduit d'échappement.

L'insert est agencé de façon que l'axe central du flux d'air comprimé s'étende selon une direction tangentielle au diamètre extérieur de la roue mobile.

Le conduit d'amenée du flux d'air comprimé se compose d'un premier et d'un second conduit rectilignes disposés dans le prolongement l'un de l'autre, le second conduit rectiligne présentant un axe longitudinal de symétrie qui s'étend selon une direction tangentielle au diamètre extérieur de la roue mobile.

Les moyens d'injection de la pièce à main se présentent sous la forme d'un insert destiné à être monté à l'intérieur du manche de la pièce à main. Le fait de disposer d'une pièce rapportée permet de canaliser le flux d'air selon une direction tangente au diamètre extérieur de la roue mobile plus facilement que dans le cas où le conduit d'amenée de l'air est usiné par des techniques classiques dans le corps de la pièce à main. En effet, dans les pièces à main à air comprimé, la turbine est agencée dans une tête qui est typiquement disposée dans le prolongement d'un manche de préhension généralement rectiligne à l'intérieur duquel est usiné le conduit d'amenée de l'air comprimé. Sauf à prendre des mesures complexes et donc économiquement peu viables, il n'est pas possible d'usiner des portions coudées par des techniques classiques dans le manche de préhension de la pièce à main. Par conséquent, le conduit d'amenée de l'air comprimé doit être usiné de biais par rapport à l'axe longitudinal de symétrie du manche de préhension si l'on souhaite que le flux d'air vienne frapper les aubes de la turbine selon une direction qui se rapproche de la tangente au diamètre extérieur de la roue mobile. Or, il faut également pouvoir usiner dans le manche de préhension de la pièce à main un conduit d'échappement par lequel s'échappe l'air après avoir frappé les aubes de la roue mobile. L'inclinaison du conduit d'amenée de l'air comprimé par rapport à l'axe longitudinal de symétrie du manche de préhension est donc nécessairement limitée car, dans le cas contraire, le conduit d'amenée de l'air comprimé déboucherait dans le conduit d'échappement, ce qui est techniquement inenvisageable.

L'insert qui sert à forcer le flux d'air comprimé en direction des aubes de la roue mobile est monté à l'intérieur du conduit d'échappement ménagé dans le corps de la pièce à main et par lequel s'échappe l'air après avoir frappé les aubes de la roue mobile. Un tel agencement facilite le retour d'air et favorise donc grandement la chute de pression entre la pression de l'air comprimé avant qu'il ne frappe les aubes de la turbine et la pression de l'air lorsqu'il s'échappe de la tête de la pièce à main après avoir frappé les aubes de la turbine. Or, plus cette chute de pression est importante, meilleure est la conversion entre l'énergie pneumatique de l'air comprimé et l'énergie cinétique de la turbine. Cette chute de pression est encore amplifiée par le fait que l'insert est monté de manière coaxiale à l'intérieur du conduit d'échappement. Le diamètre du conduit d'échappement peut donc être augmenté, ce qui permet de ramener la pression de l'air dans la tête de la pièce à main à des valeurs proches de la pression atmosphérique. Enfin, l'efficacité de conversion entre l'énergie pneumatique et l'énergie cinétique est encore améliorée du fait que l'écoulement de l'air se fait avec le moins de turbulences et donc le moins de pertes possibles. D'autre part, les bruits de fonctionnement de la pièce à main sont réduits et on évite pratiquement toute fuite d'air dans la bouche du patient.

Les moyens d'injection forcent le flux d'air comprimé de façon que le flux d'air comprimé vienne frapper les aubes de la roue mobile de la turbine selon une direction tangente au diamètre extérieur de la roue mobile. De la sorte, on garantit que le couple exercé par le flux d'air sur les aubes de la turbine est maximum, ce qui permet d'accroître sensiblement la puissance délivrée par la turbine. A titre d'exemple, la puissance délivrée par les pièces à main à air comprimé actuelles est de l'ordre de 13 watts, alors que la puissance mesurée d'une pièce à main à air comprimé équipée d'une turbine selon l'invention est de l'ordre de 20 watts. Les praticiens disposent donc avec la pièce à main à air comprimé d'une puissance accrue, ce qui leur permet de travailler plus rapidement ou d'effectuer des soins qui, jusqu'à présent, pouvaient être difficilement réalisés à l'aide d'une pièce à main à air comprimé en raison de la trop faible puissance disponible.

D'autres caractéristiques et avantages de la présente invention ressortiront plus clairement de la description détaillée qui suit d'un mode de réalisation d'une pièce à main à air comprimé équipée d'une turbine selon l'invention, cet exemple étant donné à titre purement illustratif et non limitatif seulement en liaison avec le dessin annexé sur lequel :
- la figure 1, déjà mentionnée, est une vue en perspective et en coupe selon un plan horizontal coupant longitudinalement le manche et la tête d'une pièce à main à air comprimé selon l'art antérieur ;
- la figure 2 est une vue en coupe selon un plan horizontal coupant longitudinalement le manche et la tête d'une pièce à main à air comprimé équipée d'une turbine selon l'invention ;
- la figure 3 est une vue de côté, partiellement en transparence, de la tête et du manche de la pièce à main à air comprimé équipée d'une turbine selon l'invention ;
- la figure 4 est une représentation schématique illustrant les première et seconde surfaces d'une aube de la turbine ;
- la figure 5 illustre une variante de réalisation perfectionnée de la turbine selon l'invention ;
- la figure 6 est une vue de l'arrière de la tête de la pièce à main à air comprimé équipée d'une turbine selon l'invention, dans la zone où la tête de la pièce à main se raccorde au manche de la pièce à main ;
- la figure 7 est une vue en coupe selon un plan vertical passant par l'axe longitudinal de symétrie de la tête et d'une portion du manche de la pièce à main à air comprimé équipée d'une turbine selon l'invention, et
- la figure 8 est une représentation schématique d'une variante de réalisation de la pièce à main à air comprimé équipée d'une turbine selon l'invention.

La présente invention procède de l'idée générale inventive qui consiste à prendre toutes les mesures nécessaires pour accroître la puissance mécanique que peut délivrer une pièce à main à air comprimé. A cet effet, la présente invention cherche à optimiser l'efficacité de la conversion entre l'énergie pneumatique de l'air comprimé et l'énergie cinétique de la roue mobile de la turbine. En effet, de l'efficacité de cette conversion dépend la puissance mécanique que peut fournir la pièce à main à air comprimé. Cette efficacité de conversion est notamment étroitement liée aux conditions d'écoulement de l'air dans la tête de la turbine. Effectivement, on sait que tandis qu'une partie de l'air comprimé injecté dans la tête de la pièce à main contourne la turbine en entraînant celle-ci, une autre partie de l'air comprimé ne fait que ricocher sur les aubes de la turbine et tend donc à revenir en arrière en créant des perturbations dans la tête de la pièce à main. En prévoyant d'agencer les moyens d'échappement de l'air au moins dans la zone où cet air est injecté, l'évacuation de l'air comprimé qui ricoche sur les aubes de la turbine et qui tend à créer des turbulences dans la tête de la pièce à main est facilitée. L'efficacité de conversion entre l'énergie pneumatique et l'énergie cinétique est également liée à l'angle selon lequel l'air comprimé frappe les aubes de la turbine. C'est pourquoi, les moyens d'amenée de l'air comprimé sont réalisés sous la forme d'un insert rapporté dans la pièce à main. Le fait de disposer d'une pièce rapportée permet au constructeur de jouir d'une liberté dans le positionnement de l'insert plus grande que dans le cas où le constructeur devrait usiner un conduit d'amenée de l'air comprimé par des techniques classiques dans le manche de la pièce à main. L'insert d'amenée de l'air comprimé est placé de manière coaxiale dans le conduit d'échappement de l'air comprimé. Cette disposition limite les turbulences et facilite donc le retour d'air, ce qui favorise la chute de pression entre la pression de l'air comprimé avant qu'il ne frappe les aubes de la turbine et la pression de l'air lorsqu'il s'échappe de la tête de la pièce à main après avoir frappé les aubes de la turbine. L'efficacité de la conversion entre l'énergie pneumatique de l'air comprimé et l'énergie cinétique de la roue mobile de la turbine est ainsi également optimisée. Pour atteindre ce résultat, le flux d'air comprimé est canalisé selon une direction tangente au diamètre extérieur de la turbine de la pièce à main à air comprimé. De la sorte, le flux d'air comprimé frappe les aubes de la turbine perpendiculairement à leur surface, ce qui permet de garantir que le couple exercé par le flux d'air sur les aubes de la turbine est maximum et donc d'accroître sensiblement la puissance délivrée par la turbine. Enfin, les aubes de la turbine selon l'invention se composent chacune d'une première surface qui s'étend depuis le diamètre extérieur de la roue mobile et mène jusqu'à une seconde surface qui s'étend selon un rayon de la roue mobile en s'éloignant du centre de la roue mobile. L'air comprimé tombe donc perpendiculairement sur la seconde surface de l'aube et est refoulé vers l'arrière en étant dévié de 180°. Or, plus l'angle de déviation du flux d'air comprimé est important, plus la force exercée sur les aubes de la turbine est importante. Enfin, les bruits de fonctionnement de la turbine selon l'invention sont limités et l'on travaille quasiment à la pression atmosphérique, de sorte que très peu d'air fuie dans la bouche du patient.

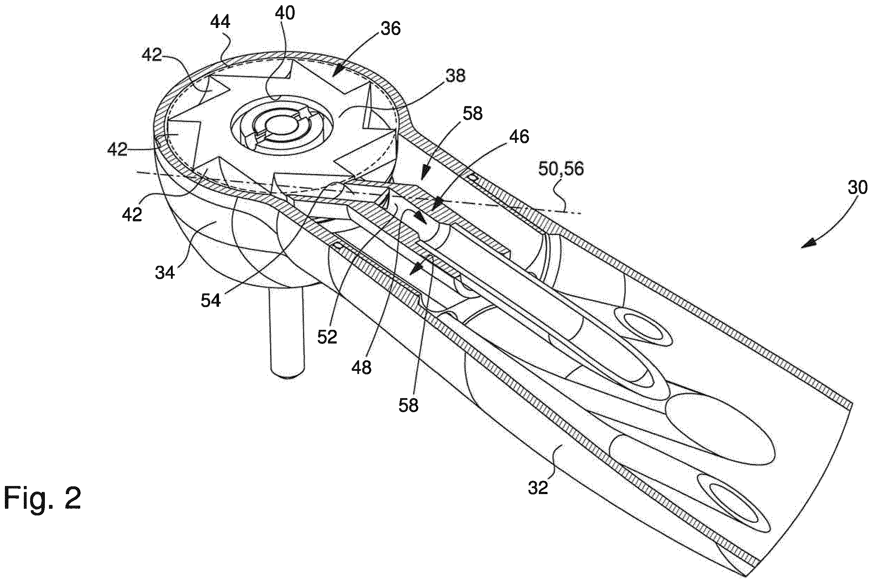

La figure 2 est une vue en coupe selon un plan horizontal coupant longitudinalement le manche et la tête d'une pièce à main à air comprimé équipée d'une turbine selon l'invention. Désignée dans son ensemble par la référence numérique générale 30, la pièce à main comprend un manche 32 qui se raccorde à une tête 34 à l'intérieur de laquelle est logée une turbine 36 selon l'invention. La turbine 36 comprend une roue mobile 38 depuis une périphérie 40 de laquelle s'étend une pluralité d'aubes 42 qui définissent un diamètre extérieur 44 de la roue mobile 38.

La pièce à main 30 comprend également des moyens d'injection ayant pour fonction de diriger sur les aubes 42 de la roue mobile 38 un flux d'air comprimé dont l'énergie pneumatique se transforme en énergie cinétique lorsque le flux d'air comprimé frappe les aubes 42 de la roue mobile 38.

A cet effet, les moyens d'injection de l'air comprimé se présentent sous la forme d'un insert 46 indépendant monté à l'intérieur de la pièce à main 30 et à l'intérieur duquel est ménagé un conduit 48 d'amenée du flux d'air comprimé. De manière préférée mais non limitative, le conduit 48 est agencé de façon que l'axe central 50 du flux d'air comprimé s'étende selon une direction tangentielle au diamètre extérieur 44 de la roue mobile 38. Les moyens d'injection forcent le flux d'air comprimé de façon que le flux d'air comprimé vienne frapper les aubes 42 de la roue mobile 38 de la turbine 36 selon une direction tangente au diamètre extérieur 44 de la roue mobile 38. De la sorte, on garantit que le couple exercé par le flux d'air sur les aubes 42 de la turbine 36 est maximum, ce qui permet d'accroître sensiblement la puissance délivrée par la turbine 36.

Le conduit 48 d'amenée du flux d'air comprimé se compose d'un premier et d'un second conduit rectiligne, respectivement 52 et 54, disposés dans le prolongement l'un de l'autre, l'axe longitudinal de symétrie 56 du second conduit rectiligne 54 s'étendant selon une direction tangentielle au diamètre extérieur 44 de la roue mobile 38.

L'insert 46 qui sert à forcer le flux d'air comprimé en direction des aubes 42 de la roue mobile 38 est monté à l'intérieur d'un conduit d'échappement 58 ménagé dans le manche 32 de la pièce à main 30 et par lequel s'échappe l'air après avoir frappé les aubes 42 de la roue mobile 38. De manière préférée mais non limitative, l'insert 46 est monté de manière coaxiale à l'intérieur du conduit d'échappement 58.

L'agencement ci-dessus favorise grandement la chute de pression entre la pression de l'air comprimé avant qu'il ne frappe les aubes 42 de la turbine 36 et la pression de l'air lorsqu'il s'échappe de la tête 34 de la pièce à main 30 après avoir frappé les aubes 42 de la turbine 36. Or, plus cette chute de pression est importante, meilleure est la conversion entre l'énergie pneumatique de l'air comprimé et l'énergie cinétique de la turbine 36. Cette chute de pression est encore amplifiée par le fait que l'insert 46 est monté de manière coaxiale à l'intérieur du conduit d'échappement 58. Le diamètre du conduit d'échappement 58 peut donc être augmenté, ce qui facilite le retour d'air et permet de ramener la pression de l'air dans la tête 34 de la pièce à main 30 à des valeurs proches de la pression atmosphérique. Enfin, l'efficacité de conversion entre l'énergie pneumatique et l'énergie cinétique est encore améliorée du fait que l'écoulement de l'air se fait avec le moins de turbulences et donc le moins de pertes possibles. D'autre part, les bruits de fonctionnement de la pièce à main 30 sont réduits et on évite pratiquement toute fuite d'air dans la bouche du patient dans la mesure où la pression dans la tête 34 de la turbine 36 est proche de la pression atmosphérique.

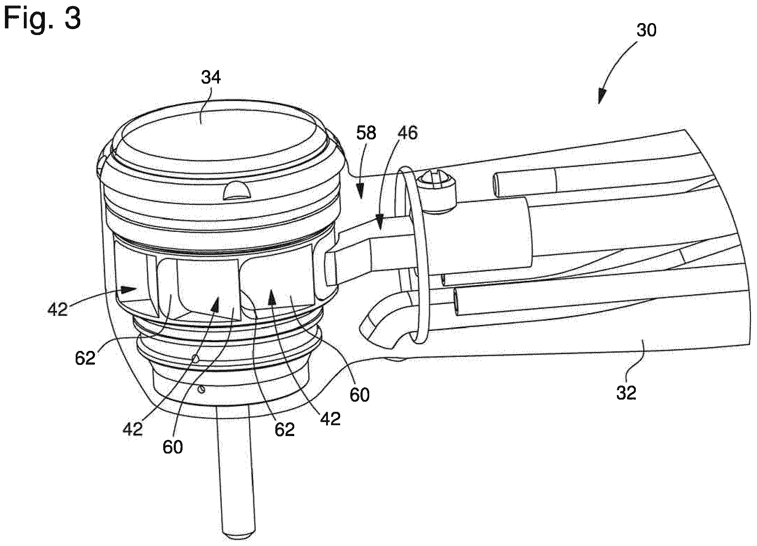

La figure 3 est une vue de côté, partiellement en transparence, de la tête 34 et du manche 32 de la pièce à main 30 à air comprimé équipée de la turbine selon l'invention. Comme on peut le voir à l'examen de cette figure, chacune des aubes 42 de la turbine 36 se compose d'une première surface 60 qui s'étend depuis le diamètre extérieur 44 de la roue mobile 38 et qui mène jusqu'à une seconde surface 62 qui s'étend radialement selon un rayon R de la roue mobile 38.

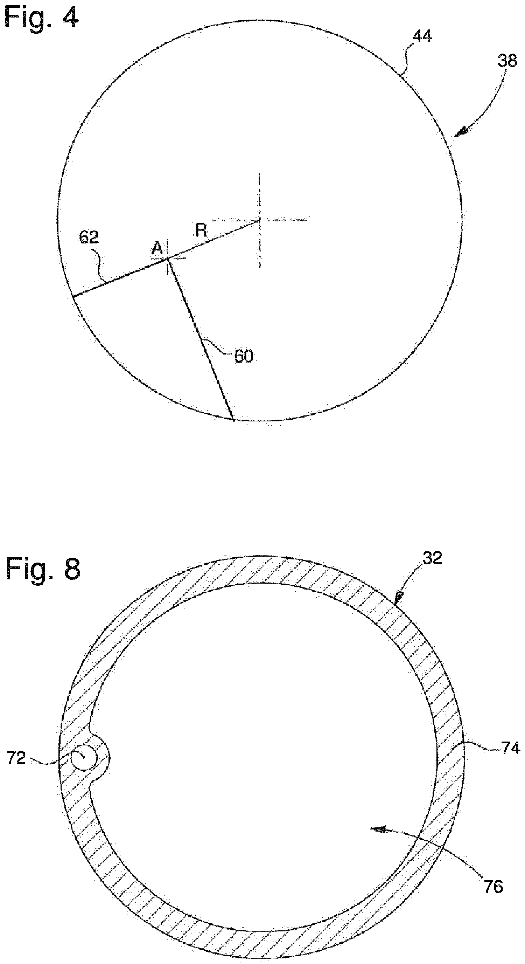

Plus précisément (voir également la figure 4), la première surface 60 s'étend depuis le diamètre extérieur 44 de la roue mobile 38 jusqu'à un point A du rayon R de la roue mobile 38 à partir duquel s'étend la seconde surface 62 jusqu'au diamètre extérieur 44 de la roue mobile 38. La première surface 60 est avantageusement plane, tandis que la seconde surface 62 présente un profil en arc de cercle tangent au rayon R de la roue mobile 38. Préférentiellement, la première surface 60 forme avec la seconde surface 62 un angle droit.

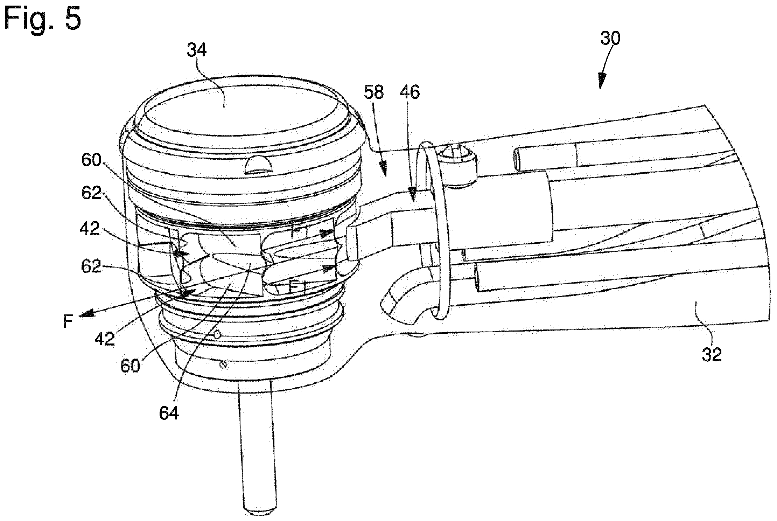

Enfin (voir figure 5), selon une variante préférée de réalisation de l'invention, les aubes 42 sont associées par paires de manière étagée, chaque aube 42 étant séparée de l'aube à laquelle elle est associée par une échancrure 64 qui s'étend dans la direction du flux d'air comprimé F, de sorte que le flux d'air F se partage en deux parts égales F1 au moment où il atteint l'échancrure 64. Cette échancrure 64 a pour but de réduire le temps de passage d'une aube à l'aube suivante afin de limiter les pertes de puissance mécanique de la turbine 36.

De ce qui précède, on comprend que la première surface 60 des aubes 42 conduit vers la seconde surface 62 l'air comprimé qui sort des moyens d'injection. Comme la seconde surface 62 s'étend selon un rayon R de la roue mobile 38 et que le flux d'air comprimé est canalisé selon une direction tangente au diamètre extérieur 44 de la roue mobile 38, le flux d'air comprimé tombe perpendiculairement sur la seconde surface 62 de l'aube 42 puis fait un demi-tour en étant défléchie par le profil en arc de cercle de la seconde surface 62. Or, la puissance mécanique délivrée par une turbine est d'autant plus grande que l'angle de déviation de l'air comprimé est important. Dans le cas de la présente invention, l'angle de déviation de l'air comprimé est de 180°, ce qui correspond à un optimum de conversion entre l'énergie pneumatique de l'air comprimé et l'énergie cinétique de la turbine 36.

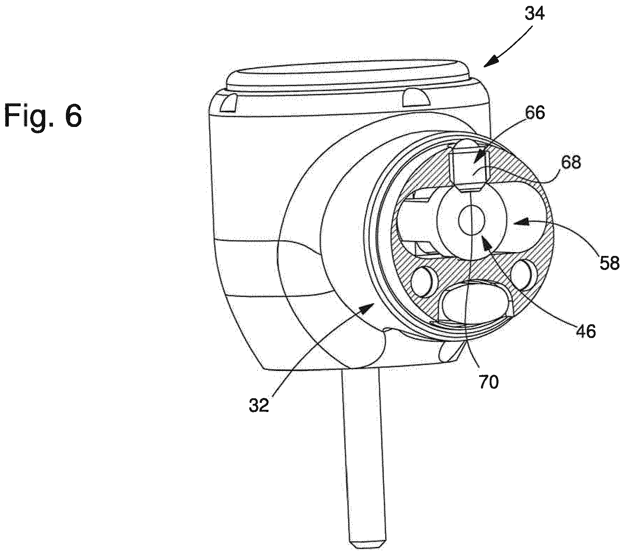

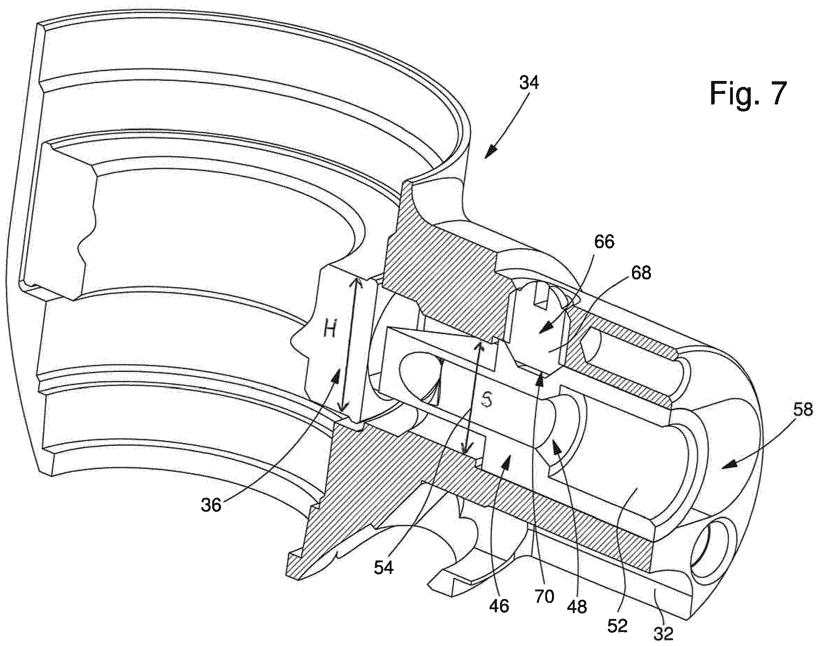

La figure 6 est une vue de l'arrière de la tête 34 de la pièce à main 30 à air comprimé équipée d'une turbine selon l'invention, dans la zone où la tête 34 de la pièce à main 30 se raccorde au manche 32 de la pièce à main 30, et la figure 7 est une vue en coupe selon un plan vertical passant par l'axe longitudinal de symétrie de la tête 34 et d'une portion du manche 32 de la pièce à main 30 à air comprimé équipée d'une turbine selon l'invention. Il ressort notamment clairement de ces deux figures que l'insert 46 qui sert à forcer le flux d'air comprimé en direction des aubes 42 de la roue mobile 38 est monté à l'intérieur du conduit d'échappement 58 ménagé dans le manche 32 de la pièce à main 30 et par lequel s'échappe l'air après avoir frappé les aubes 42 de la roue mobile 38. Préférentiellement, l'insert 46 est monté de manière coaxiale à l'intérieur du conduit d'échappement 58. De la sorte, le conduit d'échappement 58 est le plus grand possible et se trouve aussi près que possible du conduit 48 d'amenée du flux d'air comprimé, ce qui facilite le retour d'air et permet de maximiser la chute de pression, de sorte que la conversion entre l'énergie pneumatique de l'air comprimé et l'énergie cinétique de la turbine 36 est optimisée. On observe également à l'examen de la figure 7 que la section S du conduit d'échappement 58 s'étend sur au moins la hauteur H des aubes 42 de la roue mobile 38.

L'insert 46 comprend des moyens pour le réglage de sa position à l'intérieur du manche 32 de la pièce à main 30. Selon un exemple donné à titre purement illustratif et non limitatif seulement, les moyens de réglage comprennent une vis de réglage 66 dont un pied 68 est reçu dans un siège 70 ménagé à la périphérie de l'insert 46. Le siège 70 pourrait être remplacé par deux plats.

Il va de soi que la présente invention n'est pas limitée au mode de réalisation qui vient d'être décrit, et que diverses modifications et variantes simples peuvent être envisagées par l'homme du métier sans sortir du cadre de l'invention tel que défini par les revendications annexées. Les moyens d'injection de l'air comprimé se présentent préférentiellement sous la forme d'un insert 46 indépendant monté à l'intérieur de la pièce à main 30 et à l'intérieur duquel est ménagé un conduit 48 d'amenée du flux d'air comprimé. Cette solution a pour but de surmonter les problèmes que rencontrerait l'homme du métier s'il cherchait à usiner de biais un conduit d'amenée de l'air comprimé par des techniques classiques dans le manche par exemple en acier inoxydable d'une pièce à main. Une autre solution existe cependant. En effet, dans le cas où la pièce à main est réalisée par moulage d'un matériau tel qu'une céramique, il peut être envisageable de concevoir les moules de fabrication de façon que le conduit d'amenée s'étende de manière coaxiale à l'intérieur du conduit d'échappement. Il est également envisageable de réaliser la pièce à main par injection d'un matériau plastique ou d'un matériau métallique, cette dernière technique étant mieux connue sous sa dénomination anglo-saxonne Metal Injection Moulding ou MIM.

Selon une variante de réalisation, les moyens 72 d'injection de l'air comprimé sont ménagés dans la paroi 74 du manche 32 de la pièce à main 30, et les moyens 76 d'échappement de l'air comprimé occupent la majeure partie de la section du manche 32. Un tel agencement peut typiquement être obtenu par les techniques dites d'impression en trois dimensions.

## Revendications

1. Turbine pour une pièce à main à air comprimé à usage dentaire ou chirurgical, la turbine (36) comprenant une roue mobile (38) depuis une périphérie (40) de laquelle s'étendent une pluralité d'aubes (42) qui définissent un diamètre extérieur (44) de la roue mobile (38) et sur lesquelles tombe un flux (F) d'air comprimé, chacune des aubes (42) se composant d'une première surface (60) qui s'étend depuis le diamètre extérieur (44) de la roue mobile (38) et qui mène jusqu'à une seconde surface (62) qui s'étend selon un rayon (R) de la roue mobile (38), les aubes (42) étant associées par paires de manière étagée, **caractérisée en ce que** chaque aube (42) est séparée de l'aube à laquelle elle est associée par une échancrure (64) qui s'étend dans la direction du flux d'air comprimé (F), de sorte que le flux d'air comprimé (F) se partage en deux parts égales (F1) au moment où il atteint l'échancrure (64), l'échancrure (64) permettant de réduire un temps de passage du flux d'air comprimé d'une aube à l'autre.

2. Turbine pour une pièce à main à air comprimé selon la revendication 1, **caractérisée en ce que** la première surface (60) s'étend depuis le diamètre extérieur (44) de la roue mobile (38) jusqu'à un point (A) du rayon (R) de la roue mobile (38) à partir duquel s'étend radialement la seconde surface (62) jusqu'au diamètre extérieur (44) de la roue mobile (38).

3. Turbine pour une pièce à main à air comprimé selon l'une des revendications 1 ou 2, **caractérisée en ce que** la première surface (60) est plane, tandis que la seconde surface (62) présente un profil en arc de cercle tangent au rayon (R) de la roue mobile (38).

4. Turbine pour une pièce à main à air comprimé selon l'une des revendications 1 à 3, **caractérisée en ce que** la pluralité d'aubes (42) comprend au moins deux aubes adjacentes, la seconde surface (62) de l'une de ces aubes adjacentes se terminant à l'endroit où débute la première surface (60) de l'autre aube adjacente.

5. Turbine pour une pièce à main à air comprimé selon l'une des revendications 1 à 4, **caractérisée en ce que** la première surface (60) formant un angle droit avec la seconde surface (62).

## Patentansprüche

1. Turbine für ein Drucklufthandstück zur zahnärztlichen oder chirurgischen Verwendung, wobei die Turbine (36) ein Laufrad (38) umfasst, von dessen Umfang (40) sich mehrere Radschaufeln (42) erstrecken, die einen Außendurchmesser (44) des Laufrades (38) definieren, und auf die ein Druckluftfluss (F) fällt, wobei jede der Schaufeln (42) eine erste Fläche (60) besitzt, die sich vom Außendurchmesser (44) des Laufrades (38) aus erstreckt und die bis zu einer zweiten Fläche (62) führt, die sich entlang einem Radius (R) des Laufrades (38) erstreckt, wobei die Schaufeln (42) paarweise stufenartig verbunden sind, **dadurch gekennzeichnet, dass** jede Schaufel (42) von der Schaufel, mit der sie verbunden ist, durch eine Einbuchtung (64) getrennt ist, die sich in der Richtung des Druckluftflusses (F) erstreckt, so dass der Druckluftfluss (F) sich in zwei gleiche Teile (F1) in dem Moment aufteilt, in dem er die Einbuchtung (64) erreicht, wobei die Einbuchtung (64) die Verringerung der Durchflusszeit des Druckluftflusses von einer Schaufel zur anderen ermöglicht.

2. Turbine für ein Drucklufthandstück nach Anspruch 1, **dadurch gekennzeichnet, dass** die erste Fläche (60) sich vom Außendurchmesser (44) des Laufrades (38) bis zu einem Punkt (A) des Radius (R) des Laufrades (38) erstreckt, von dem aus sich die zweite Fläche (62) radial bis zum Außendurchmesser (44) des Laufrades (38) erstreckt.

3. Turbine für ein Drucklufthandstück nach Anspruch 1 oder 2, **dadurch gekennzeichnet, dass** die erste Fläche (60) eben ist, während die zweite Fläche (62) ein Kreisbogenprofil tangential zum Radius (R) des Laufrades (38) aufweist.

4. Turbine für ein Drucklufthandstück nach einem der Ansprüche 1 bis 3, **dadurch gekennzeichnet, dass** die mehreren Schaufeln (42) mindestens zwei benachbarte Schaufeln umfassen, wobei die zweite Fläche (62) einer dieser benachbarten Schaufeln an der Stelle endet, an der die erste Fläche (60) der anderen benachbarten Schaufel beginnt.

5. Turbine für ein Drucklufthandstück nach einem der Ansprüche 1 bis 4, **dadurch gekennzeichnet, dass** die erste Fläche (60) zu der zweiten Fläche (62) einen rechten Winkel bildet.

## Claims

1. Turbine for a compressed air handpiece for dental or surgical use, the turbine (36) including a moving wheel (38) from a periphery (40) of which extend a plurality of blades (42) which define an outer diameter (44) of the moving wheel (38) and on which a compressed air flow (F) falls, each of the blades (42) being formed of a first surface (60) which extends from the outer diameter (44) of the moving wheel (38) and which leads to a second surface (62) which extends along a radius (R) of the moving wheel (38), the blades (42) being associated in pairs in a stepped manner, **characterized in that** each blade (42) is separated from the blade associated therewith by a notch (64) which extends in the direction of the compressed air flow (F), so that the compressed air flow (F) is divided into two equal parts (F1) at the moment when the flow reaches the notch (64), the notch (64) allowing a reduction of a transit time of the compressed air flow from one blade to the other.

2. Turbine for a compressed air handpiece according to claim 1, **characterized in that** the first surface (60) extends from the outer diameter (44) of the moving wheel (38) to a point (A) on the radius (R) of the moving wheel (38) from which the second surface (62) extends radially to the outer diameter (44) of the moving wheel (38).

3. Turbine for a compressed air handpiece according to claim 1 or 2, **characterized in that** the first surface (60) is plane, whereas the second surface (62) has a profile in the shape of an arc of a circle tangential to the radius (R) of the moving wheel (38).

4. Turbine for a compressed air handpiece according to any of claims 1 to 3, **characterized in that** the plurality of blades (42) comprises at least two neighbouring blades, the second surface (62) of one of these neighbouring blades ending at the location where starts the first surface (60) of the other neighbouring blade.

5. Turbine for a compressed air handpiece according to any of claims 1 to 4, **characterized in that** in the first surface (60) forms a right angle with second surface (62).
